# EUROPÄISCHE PATENTSCHRIFT

(11) **EP 0 599 325 B1**
(45) Veröffentlichungstag und Bekanntmachung des Hinweises auf die Patenterteilung: **03.03.1999**
(21) Anmeldenummer: 93119041.7
(22) Anmeldetag: 25.11.1993
(51) Int. Cl.: C07H 23/00, G01N 33/82

(54) **Verbesserte B12-Konjugate**
B12 conjugates
Conjugés de B12

(30) Priorität: 26.11.1992 DE 4239815
(43) Veröffentlichungstag der Anmeldung: 01.06.1994
(73) Patentinhaber: BOEHRINGER MANNHEIM GMBH, 68305 Mannheim (DE)
(72) Erfinder: Hoess, Eva, Dr., D-82319 Starnberg (DE); Stock, Werner, Dr., D-82166 Graefeling (DE); Huber, Erasmus, Dr., D-86923 Finning (DE)
(74) Vertreter: Huber, Bernhard, Dipl.-Chem.

(56) Entgegenhaltungen:
- EP-A- 0 361 817
- EP-A- 0 378 197
- EP-A- 0 378 203
- EP-A- 0 378 204
- WO-A-89/12826
- WO-A-91/00519

## Beschreibung

Die Erfindung betrifft ein neues Cobalamin-Konjugat, dessen Herstellung und Verwendung im Rahmen von Immuntests, insbesondere zur Bestimmung von Vitamin B₁₂.

Die immunologische Bestimmung von Cobalaminen (z.B. Vitamin B₁₂) erfolgt üblicherweise in einem kompetitiven Test, bei dem das Cobalamin der Probe und radioaktiv markiertes Cobalamin um ein an eine Festphase gebundenes Bindeprotein für Cobalamin (Intrinsic Factor) konkurrieren (RIA-Test). Nach Trennung der festen von der flüssigen Phase kann in einer der beiden Phasen aus der dort enthaltenen Menge an radioaktiver Markierung die Menge des in der Probe enthaltenen Cobalamins ermittelt werden.

Aufgrund der bekannten Nachteile von RIA-Tests (z.B. radioaktive Belastung, Entsorgung) ist es wünschenswert, die radioaktive Markierung durch eine nicht-radioaktive Markierung, z.B. durch eine enzymatische Markierung zu ersetzen.

Bekannte Kupplungstechniken zur Herstellung von Protein-Vitamin B₁₂-Konjugaten als Immunogene sind bekannt (Gershman et al., Archives of Biochemistry and Biophysics 153 (1972), 407; v.d.Weel et al., Clin.Chim.Acta 56 (1974), 143; Endres et al., Clin.Chem. 24/3 (1978), 460; US-Patent 3,981,863). Die gemäß den obigen Techniken hergestellten Protein-Vitamin B₁₂-Konjugate wurden bereits als Immunogene beschrieben. Sie sind jedoch hydrolyseempfindlich und nur als instabile Zwischenstufen formulierbar. Daher ist die Kupplungsausbeute relativ niedrig und, wenn überhaupt, nur sehr schwer reproduzierbar. Die durch die Kupplung erhaltenen Produkte sind ebenfalls nur schwer charakterisierbar. Zudem findet eine unkontrollierte Polymerisation des freien Enzyms als Nebenreaktion statt.

Die EP-A 0 378 204 offenbart Cobalamin-Säurehydrazide und Konjugate dieser Substanzen mit Glycoproteinen. Diese Konjugate können zwar auf einfache und reproduzierbare Weise hergestellt werden. Das Kupplungsprinzip kann jedoch nur bei Glycoproteinen angewendet werden, deren Hydroxylgruppen am Glycosylrest nach der Oxidation mit Perjodat zur Ausbildung einer Hydrazongruppierung zur Verfügung stehen.

Die der vorliegenden Erfindung zugrundeliegende Aufgabe bestand somit darin, neue Cobalamin-Konjugate unter zumindest weitgehender Vermeidung der Nachteile des Standes der Technik bereitzustellen, die auf einfache und reproduzierbare Weise herstellbar sind und seitens des Kupplungspartners keine Beschränkungen aufweisen.

Weiterhin sollte die Kupplung von Cobalamin an den Kupplungspartner aufgrund des hohen Preises von Cobalaminen in guten Ausbeuten und mit einer akzeptablen Reaktionsdauer erfolgen.

Diese Aufgabe wird gelöst durch ein Verfahren zur Herstellung von Cobalamin-Konjugaten der Formel (I)

B - CO - Sp - P (I)

wobei B den aus einem Cobalamin durch Abspaltung einer -CONH₂ Gruppe gebildeten Rest,
P den Kupplungspartner des Cobalamins und Sp eine Spacergruppierung darstellt, welches dadurch gekennzeichnet ist, daß man eine Cobalamin-Monocarbonsäure B-COOH mit einem Chlorameisensäureester der allgemeinen Formel (II) 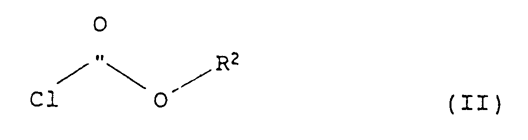 als Kondensationsmittel zur Kupplung umsetzt, worin R² einen geradkettigen oder verzweigten Alkylrest mit 1 bis 6 Kohlenstoffatomen darstellt, wobei eine aktivierte Cobalamin-Monocarbonsäure der allgemeinen Formel (III) 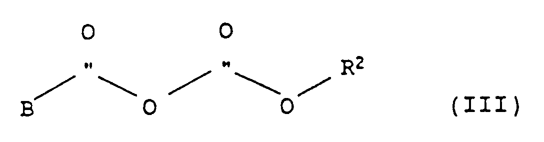 entsteht, die anschließend als Ausgangsmaterial für die Verknüpfung mit dem Kupplungspartner P über den Spacer Sp dient.

Es wurde überraschenderweise gefunden, daß die Verwendung eines Chlorameisensäureesters eine mit guten Ausbeuten und schnell durchführbare Aktivierung des Cobalamins bewirkt, während andere Kupplungsreagenzien nur unbefriedigende Resultate liefern.

Bevorzugt umfaßt das erfindungsgemäße Verfahren weiterhin die Verwendung der hergestellten Cobaldamin-Konjugate in Immuntests, insbesondere zur Bestimmung von Cyanocobalamin.

Ein weiterer Gegenstand der Erfindung ist ein Cobalamin-Konjugat, welches durch das erfindungsgemäße Verfahren erhältlich ist. Vorzugsweise besitzt das erfindungsgemäße Cobalamin-Konjugat die allgemeine Formel (XVII) 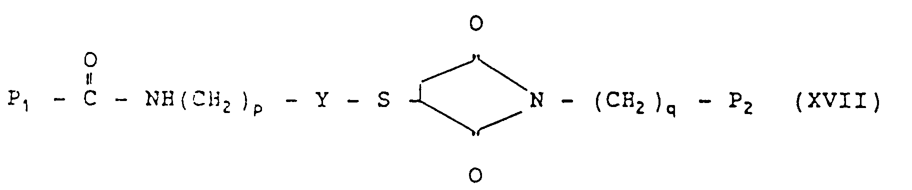 worin
entweder P₁ einen aus einem Cobalamin durch Abspaltung einer -CONH₂-Gruppe gebildeten Rest und P₂ den Kupplungspartner des Cobalamins darstellt,
oder P₂ einem aus einem Cobalamin durch Abspaltung eines H-Atoms (Amidwasserstoff) gebildeten Rest und P₁ den Kupplungspartner des Cobalamins darstellt, p und q gleich oder verschieden und ganze Zahlen von 2 bis 6 sind, und
Y eine Einfachbindung oder eine Gruppe der allgemeinen Formel (XVIII) 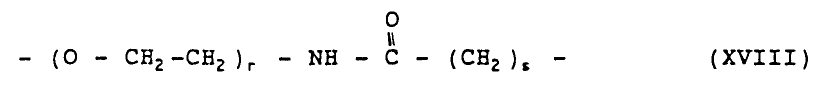 ist, wobei r eine ganze Zahl von 0 bis 3 und s eine ganze Zahl von 1 bis 3 ist.

Die in der allgemeinen Formel (XVII) zwischen P₁ und P₂ befindliche Gruppierung ist somit ein bevorzugter Spacer Sp für ein Verfahren zur Herstellung von Cobalamin-Konjugaten der Formel (I).

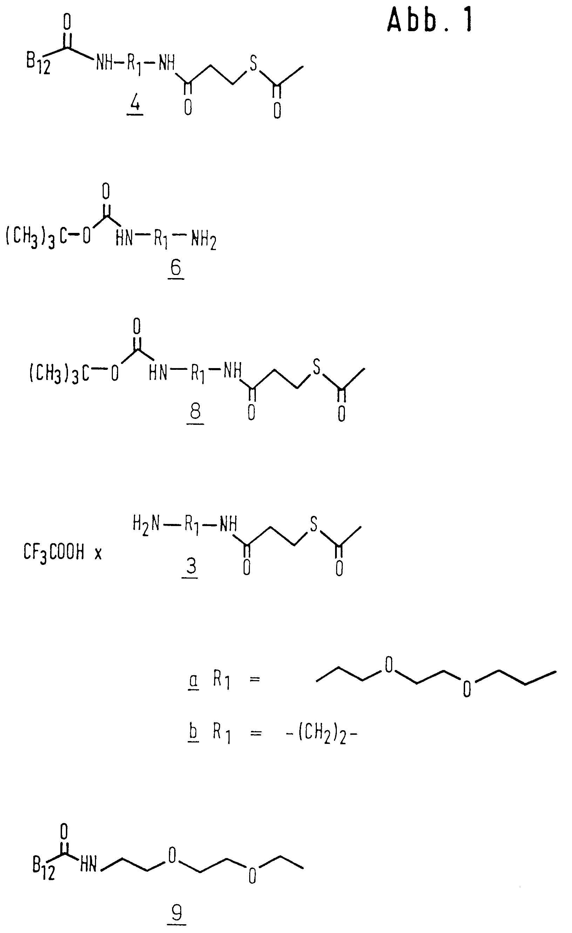

Für Verbindungen der Formel (XVII) ist es bevorzugt, wenn p gleich 2 und q gleich 2 oder 6 sind. Weiterhin ist es bevorzugt, daß wenn Y eine Gruppe der Formel (XVIII) ist, r 0 oder 2 ist. Ferner ist s vorzugsweise gleich 2, wenn Y eine Gruppe der Formel (XVIII) ist. Besonders bevorzugt sind auch p, q, r und s gleich 2 (Verbindungen "a" in Abb. 1) oder p, q und s gleich 2 und r gleich 0 (Verbindungen "b" in Abb. 1), wenn Y eine Gruppe der Formel (XVIII) ist. Wenn Y in Formel (XVII) eine Einfachbindung ist, ist p besonders bevorzugt gleich 2 und q gleich 2 oder 6, wobei derartige Verbindungen durch Verwendung von Cystamin als Spacerbestandteil erhältlich sind.

Das Cobalamin wird bei Verbindungen der Formel (XVIII) - unabhängig davon, ob es P₁ oder P₂ ist - in der Form einer B-CO-NH-Gruppierung eingeführt. Der Bindepartner des Cobalamins wird vorzugsweise in einer der Formen Bindepartner-CONH- oder Bindepartner-NHCO- in das Konjugat (XVII) eingeführt. Diese beide Formen sind allerdings nur möglich, wenn P₂ der Bindepartner des Cobalamins ist. Wenn hingegen bei Verbindungen der Formel (XVII) P₁ der Bindepartner des Cobalamins ist, so kann er nur als Bindepartner-CONH- eingeführt werden.

Das erfindungsgemäße Cobalamin-Konjugat enthält 3 Komponenten. Vorzugsweise besitzt dabei die Cobalamin-Komponente die allgemeine Formel (IV) 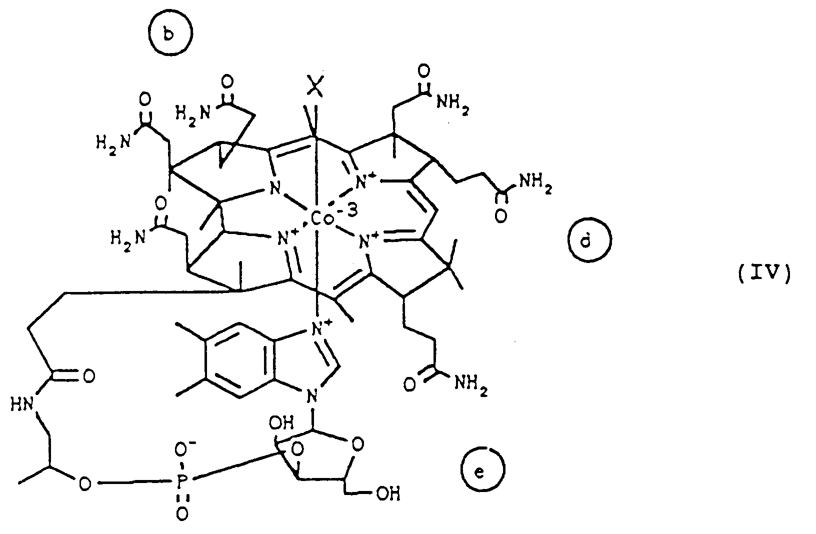 wobei X einen Liganden des Kobalts darstellt. Der Ligand X kann beispielsweise H₂O (Vitamin B_{12A}), OH (Vitamin B_{12B}), NO (Vitamin B_{12C}), eine Adenosylgruppe (Coenzym B₁₂) oder ein CN (Vitamin B₁₂) darstellen. Vorzugsweise ist X eine CN-Gruppe, so daß das Cobalamin ein Cyanocobalamin ist. Es ist aber auch möglich, daß der Ligand X fehlt.

Die Bindung des Cobalamins mit der Spacergruppierung erfolgt vorzugsweise über eine der mit (b), (d) oder (e) bezeichneten Positionen, besonders bevorzugt über eine der mit (d) oder (e) bezeichneten Positionen und am meisten bevorzugt über die mit (d) bezeichnete Position.

Eine Bindung der Spacergruppierung Sp an diese Positionen kann durch Überführung der jeweiligen in Formel (IV) gezeigten Säureamidgruppe -CONH₂ in die freie Carboxylgruppe auf an sich bekannte Weise durch saure Verseifung und Isolierung der freien Carbonsäuren (vgl. Yamada und Hogenkamp, J.Biol.Chem. 247 (1972), 6266-6270; Anton et al., J.Amer.Chem.Soc. 102 (1980), 2215) erfolgen. Vorzugsweise arbeitet man so, daß eine unvollständige Verseifung der im Cobalaminmolekül vorhandenen Säureamidgruppen erreicht wird, damit nur eine oder einige wenige der entsprechenden Carboxylgruppen freigesetzt werden. Aus dem so erhaltenen Gemisch kann dann nach an sich bekannten Methoden (z.B. Inorg.Chim.Acta 162 (1989), 151-155) die Verbindung isoliert werden, in der die freie Carboxylgruppe an einer bestimmten gewünschten Stelle vorliegt. Es ist aber auch möglich, von einem Gemisch der Cobalamincarbonsäuren auszugehen, wobei es jedoch zur Herstellung eines Cobalaminkonjugats für die Verwendung in Immuntests im Hinblick auf die Empfindlichkeit der Bestimmungsmethode zweckmäßig und vorzuziehen ist, von einer einzigen gereinigten Carbonsäure auszugehen.

Die Bindung der Spacergruppierung an das Cobalamin erfolgt über eine Säureamidgruppe, d.h. durch Verknüpfung der aktivierten Carboxylfunktion des Cobalaminderivats gemäß Formel (III) mit einer Aminofunktion des Spacers.

Eine bevorzugte Ausführungsform der Herstellung von Cobalamin-Konjugaten der Formel (I) besteht darin, daß man die aktivierte Cobalamin-Monocarbonsäure (III) mit einer Verbindung der allgemeinen Formel (V) 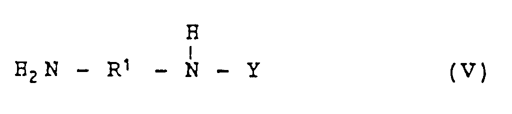 umsetzt, wobei R¹ eine unverzweigte Alkylengruppe mit 2 bis 20 Kohlenstoffatomen darstellt, die durch ein oder mehrere Heteroatome, insbesondere Sauerstoff- oder Schwefelatome, substituiert sein kann und Y Wasserstoff, eine Schutzgruppe oder eine Gruppe der allgemeinen Formel (VI) 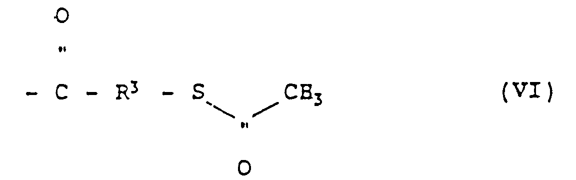 ist, worin R³ eine unverzweigte Alkylengruppe mit 1 bis 6 Kohlenstoffatomen darstellt, und das entstehende Produkt der allgemeinen Formel 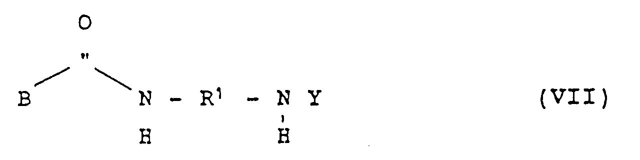 mit einem reaktiven Kupplungspartner P umsetzt, um ein Cobalamin-Konjugat der Formel (I) zu erhalten.

Vorzugsweise ist Y entweder Wasserstoff oder eine Gruppe der allgemeinen Formel (VI). Y kann jedoch auch eine Schutzgruppe, z.B. eine Benzyloxycarbonyl- oder eine tert.-Butyloxycarbonyl (BOC)-Gruppe sein, die nach der Reaktion nach bekannten Methoden wieder abgespalten werden kann.

In einer besonders bevorzugten Ausführungsform der vorliegenden Erfindung stellt R¹ eine unsubstituierte (CH₂)ₙ-Gruppe dar, wobei n eine ganze Zahl von 1 bis 5 ist. Besonders bevorzugt ist n gleich 2. In einer weiteren bevorzugten Ausführungsform stellt R¹ eine (C ₂H₄O)ₘ-(CH₂)₂-Gruppe dar, wobei m eine ganze Zahl von 1 bis 5 ist. Besonders bevorzugt ist m gleich 2. R³ ist vorzugsweise eine (CH₂ )₂-Gruppe.

Das Zwischenprodukt (VII) kann - je nach Bedeutung der Gruppe Y - anschließend auf mehrere verschiedene Arten weiterreagieren. In der ersten bevorzugten Ausführungsform wird die aktivierte Cobalamin-Monocarbonsäure (III) mit einer Verbindung der allgemeinen Formel (Va) 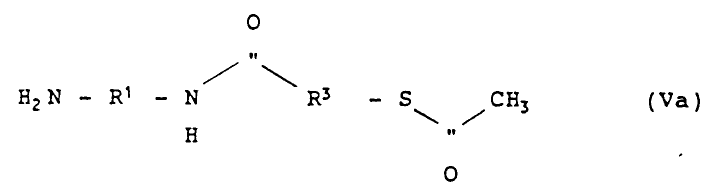 umgesetzt, worin R¹ wie in Formel (V) definiert ist und R³ eine unverzweigte Alkylengruppe mit 1 bis 6, vorzugsweise 2 Kohlenstoffatomen darstellt. Die Verbindung (Va) kann beispielsweise durch Reaktion eines Diamins H₂NR¹H₂N nach Schutz einer Aminofunktion (z.B. durch eine BOC-Schutzgruppe) und anschließender Reaktion mit N-Succinimidyl-S-acetylthiopropionsäure (SATP) synthetisiert werden. Das entstehende Produkt der allgemeinen Formel (VIII) 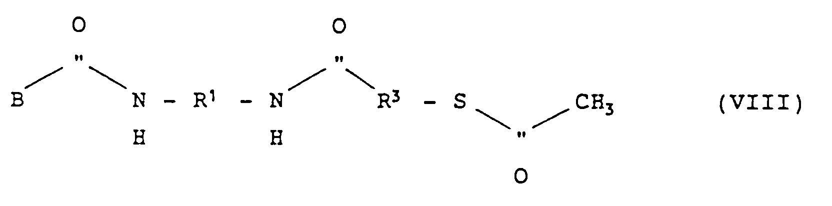 wird zur Abspaltung der S-Acetylgruppe mit Hydroxylamin behandelt und das resultierende Produkt wird mit einem Maleimid-funktionalisierten Kupplungspartner der allgemeinen Formel (IX) 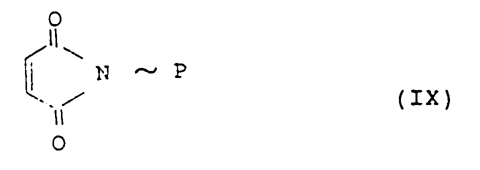 umgesetzt, wobei als Endprodukt ein Cobalamin-Konjugat der allgemeinen Formel (X) 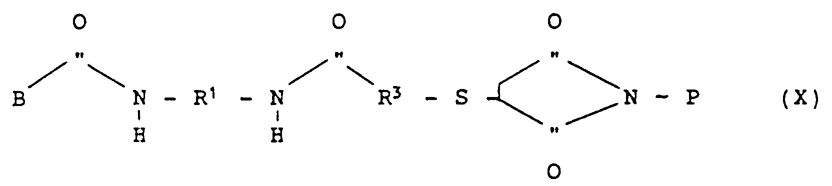 entsteht.

Der Maleimid-funktionalisierte Kupplungspartner (IX) kann beispielsweise ein Protein sein, das auf an sich bekannte Weise durch Umsetzung mit einem Maleimid-Derivat aktiviert worden ist (z.B. Yoshitake, Eur.J.Biochem. 101 (1979), 395-399).

In einer weiteren Ausführungsform des erfindungsgemäßen Verfahrens kann der Aufbau der Spacergruppierung direkt am Cobalamin erfolgen. Hierzu wird die aktivierte Cobalamin-Monocarbonsäure (III) mit einem nicht funktionalisierten Diamin der allgemeinen Formel (Vb)

H₂N - R¹ - NH₂ (Vb)

umgesetzt, wobei R¹ gemäß Formel (V) definiert ist. Diese Umsetzung wird vorzugsweise mit einem Überschuß des Amins durchgeführt, so daß ein Molekül Cobalamin mit einem Molekül (Vb) reagiert. Das entstehende Produkt (XI) 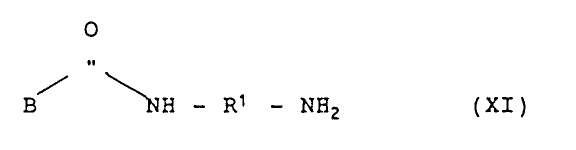 wird anschließend mit einer Verbindung der allgemeinen Formel (XII), vorzugsweise mit N-Succinimidyl-S-acetylthiopropionat (R³ = (CH₂)₂) 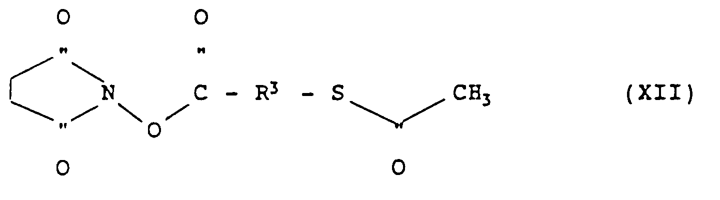 umgesetzt, wobei R³ gemäß Formel (V) definiert ist. Durch diese Reaktion entsteht ein Produkt der allgemeinen Formel (VIII) (siehe oben), das mit einem Maleimid-funktionalisierten Kupplungspartner der Formel (IX) zu einem Cobalamin-Konjugat der Formel (X) umgesetzt werden kann.

Ein Beispiel für eine geeignete Verbindung der allgemeinen Formel (Vb) ist Cystamin (2,2'-Dithiobis(ethyl-amin)). Das durch Reaktion der Cobalamin-Monocarbonsäure (III) mit Cystamin gebildete Zwischenprodukt (VII) kann anschließend an der Disulfidbrücke, beispielsweise wie in Synthesis Commun. (1974), 59, J.Org.Chem. 56 (1991), 2648-2650 oder Biochemistry 3 (1964), 480-482 beschrieben, gespalten und an einen aktivierten Kupplungspartner, vorzugsweise einen Maleimid-funktionalisierten Kupplungspartner gekuppelt werden.

Bei den bisher genannten Verfahrensvarianten wurde ein Maleimid-funktionalisierter Kupplungspartner eingesetzt. Es ist jedoch auch, ein Maleimid-funktionalisiertes Cobalamin-Derivat zu verwenden. Hierzu wird eine aktivierte Cobalamin-Monocarbonsäure (III) mit einem Maleimid-Derivat der allgemeinen Formel (XIII) 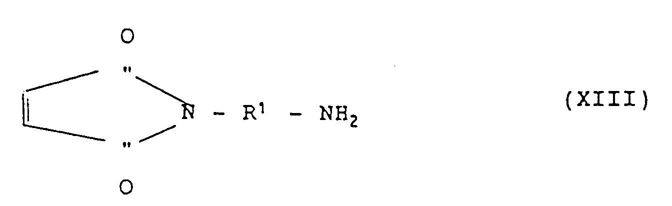 umgesetzt, wobei R¹ wie in Formel (V) definiert ist. Das entstehende Produkt der allgemeinen Formel (XIV) 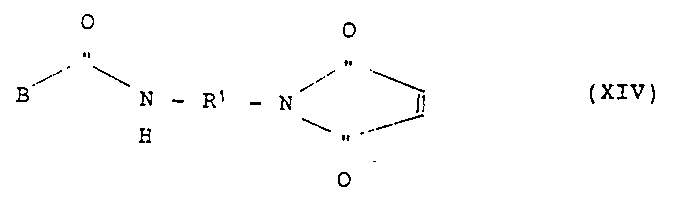 kann anschließend mit der SH-Funktion eines Thiol-funktionalisierten Kupplungspartners HS ∼ P umgesetzt werden, wobei als Endprodukt ein Cobalamin-Konjugat der allgemeinen Formel (XV) 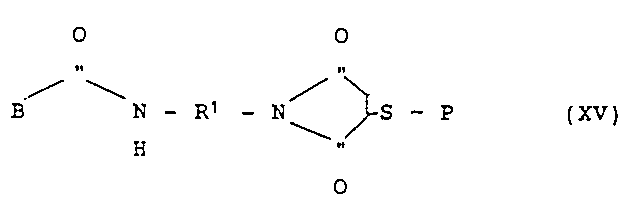 entsteht.

Bei dieser Ausführungsform des erfindungsgemäßen Verfahrens besitzt der Thiol-funktionalisierte Kupplungspartner HS ∼ P vorzugsweise die allgemeine Formel (XVI) 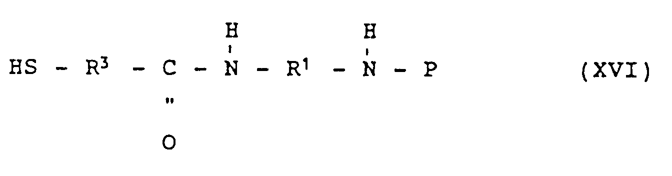 wobei R¹ und R³ gemäß Formel (V) definiert sind. Ein Beispiel für einen bevorzugten Thiol-funktionalisierten Kupplungspartner ist Biotin-DADOO-(S)ATP (XVII):

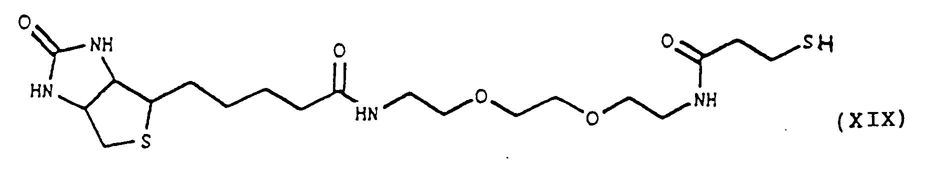

Für alle oben genannten Herstellungsverfahren ist es besonders bevorzugt, wenn R³ eine (CH₂-CH₂)-Gruppe darstellt. Weiterhin ist es bevorzugt, wenn R¹ eine (CH₂-CH₂)-Gruppe oder eine ((C₂H₄O)₂-CH₂-CH₂)-Gruppe darstellt.

Die dritte Komponente des erfindungsgemäßen Konjugats ist der Kupplungspartner P. Der Kupplungspartner P ist vorzugsweise ein nachweisbares Molekül, d.h. er enthält eine Komponente, die immunologisch, durch Affinitätsbindung (z.B. Biotin-Streptavidin bzw. Avidin, Protein A-γ Immunglobulin, Zucker-Lectin, Antikörper-Concavalin), enzymatisch, durch Fluoreszenz, Lumineszenz oder Kernresonanz nachweisbar ist. Vorzugsweise ist der Kupplungspartner immunologisch, enzymatisch oder durch Affinitätsbindung nachweisbar.

In einer bevorzugten Ausführungsform ist der Kupplungspartner P ein Protein. Dabei kann es sich um ein enzymatisch aktives Protein, ein immunologisch nachweisbares Protein (z.B. einen Antikörper oder ein Antikörperfragment) oder ein durch Affinitätsbindung nachweisbares Protein handeln. Als enzymatisch aktives Protein wird vorzugsweise alkalische Phosphatase, Peroxidase oder β-Galactosidase, besonders bevorzugt polymerisierte Peroxidase verwendet. Die Herstellung geeigneter polymerisierter Enzyme oder Polypeptide ist beispielsweise aus der EP-A 0 175 560 bekannt. Dabei werden mindestens zwei Enzymmoleküle verbunden und dieses polymerisierte Enzym dann an einen Antikörper oder ein Fragment davon gekoppelt. Die Polymerisation der Enzyme erfolgt dabei bevorzugt unter Verwendung eines Vernetzungsreagenz, das ein bifunktionelles Mittel (z.B. Glutardialdehyd) ist. Dazu werden die beiden zu vernetzenden Partner chemisch über in der jeweiligen Proteinstruktur enthaltene funktionelle Gruppen aktiviert und dann mit dem bifunktionellen Mittel gekoppelt.

Als ein durch Affinitätsbindung nachweisbares Protein wird vorzugsweise Streptavidin, Avidin oder ein Derivat davon, gegebenenfalls in polymerisierter Form verwendet.

In einer anderen bevorzugten Ausführungsform ist der Kupplungspartner ein immunologisch oder durch Affinitätsbindung nachweisbares Hapten. Beispiele für bevorzugte immunologisch nachweisbare Haptene sind Digoxigenin (oder Derivate davon), Fluorescein, p-Nitrophenol, Saponin etc.. Ein Beispiel für ein durch Affinitätsbindung nachweisbares Hapten ist Biotin oder ein Derivat davon.

Ein weiterer Gegenstand der vorliegenden Erfindung ist die Verwendung der erfindungsgemäßen Cobalamin-Konjugate in Immuntests, wie etwa ELISA, insbesondere zur Bestimmung von Cyanocobalamin (Vitamin B₁₂). Die erfindungsgemäßen Konjugate können beispielsweise zur Vitamin B₁₂-Bestimmung nach dem in der DE-OS 39 00 650 beschriebenen Verfahren verwendet werden.

Es ist jedoch auch möglich, die erfindungsgemäßen Cobalamin-Konjugate in anderen Verfahren zur Bestimmung von Vitamin B₁₂ (oder anderen Cobalaminen) anstelle eines radioaktiv markierten Cobalamins einzusetzen. Derartige geeignete Radioimmuntests sind beispielsweise in Clin.Biochem. 18 (1985), 261-266, US-PS 3,981,863, Clin.Chim.Acta 56 (1974), 143-149, Lit.Clin.Path. 20 (1967), 683-686, Brit.J.Hemat. 22 (1972), 21-31 beschrieben.

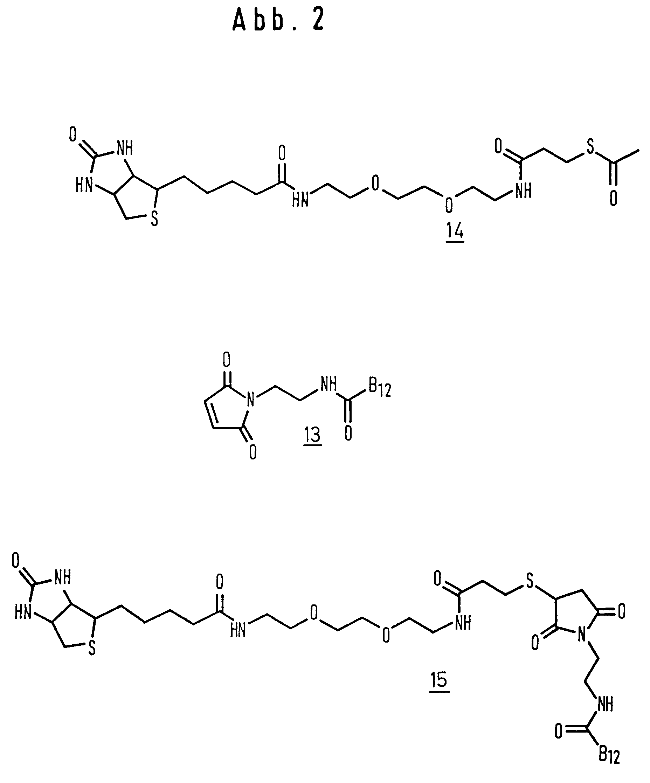

Die nachfolgenden Beispiele und Abbildungen 1 bis 3 erläutern die Erfindung weiter.

Abbildung 1 zeigt die chemischen Strukturformeln für die in den Beispielen 1 bis 11 hergestellten Verbindungen (4a), (4b), (6a), (6b), (8a), (8b), (3a), (3b) und (9).

Abbildung 2 zeigt die chemische Struktur der in den Beispielen 12 bis 14 hergestellten Verbindungen (14), (13) und (15).

Abbildung 3 zeigt Eichkurven für die B₁₂-Bestimmung, die unter Verwendung eines erfindungsgemäßen Konjugats und eines Konjugats des Standes der Technik erhalten wurden.

### Beispiel 1:

### B₁₂-d-DADOO-(S)ATP (4a)

500 mg (0.37 mmol) B₁₂-d-Säure werden in 30 ml absoluten DMF und 5 ml DMSO gelöst und mit 155 µl (1.09 mmol) Triethylamin versetzt. Nach 20 min Rühren bei 20°C wird das Reaktionsgemisch auf 0°C gekühlt und mit 143 µl (1.09 mmol) Chlorameisensäureisobutylester versetzt. Nach weiteren 15 min unter Eiskühlung wird eine Lösung aus 645.4 mg (1.4 mmol) DADOO-(S)ATP x Trifluoressigsäure (siehe Beispiel 8), 193 µl (1.4 mmol) Triethylamin und 10 ml absoluten DMF zugegeben. Dabei ist eine leichte Gasentwicklung zu beobachten. Es wird noch 30 min bei 0°C weitergerührt, anschließend bringt man das Reaktionsgemisch langsam auf 20°C, prüft mittels HPLC den Umsatz und dampft das Lösungsmittel am Hochvakuum (HV) ab (Badtemp. nicht über 50 °C). Der Rückstand wird in 25 ml Wasser gelöst und über eine Amberlite®XAD 2-Säule (3.5 x 30 cm) entsalzt. Nach dem Eluieren des Produkts mit Methanol, entfernt man das Lösungsmittel im Wasserstrahlvakuum (WSV), löst den Rückstand in 25 ml Wasser und tragt diese Lösung auf eine Dowex®1x2-Säule (Acetat-Form, 3.0 x 30 cm) auf. Das Produkt wird mit Wasser eluiert und anschließend über präparative HPLC aufgereinigt. Die sauberen Fraktionen werden gepoolt und lyophilisiert.
- Ausbeute:: 148 mg (25 %) B₁₂-d-DADOO-(S)ATP

### Analytische HPLC:

- Säule:: Vydac®C18 MZ-Micro, 300 Å, 5 µm, 4.6 x 250 mm
- Eluent:: A: 0.01 m NH₄Ac pH 6.7 B: 95 % Acetonitril 0.01 m NH₄Ac pH 6.7
- Gradient:: 0 → 80 % B in 60 min
- Fluß:: 1 ml/min
- Detektor:: UV-Detektor, Wellenlänge 226 nm

### Präparative HPLC:

- Säule:: Waters Delta-PAK™ C18, 100 Å, 15 µm, 50 x 300 mm
- Eluent:: A: 0.01 NH₄Ac pH 6.7 B: 95 % Acetonitril 0.01 mol/l NH₄Ac pH 6.7
- Gradient:: 0 % B 10 min 0 → 40 % B in 110 min 40 → 80 % B in 10 min 80 % B 20 min
- Fluß:: 15 ml/min
- Detektor:: UV-Detektor, Wellenlänge 226 nm

### Analytische Daten:

- Retentionszeit analytische HPLC:: 22.3 min
- IR (KBr):: γ = 3350, 3170, 2120, 1660, 1570, 1495, 1398 cm⁻¹
- ¹H-NMR (D₂O, TMS-Na-Salz):: enthält die Signale der B₁₂-d-Carbonsäure sowie 2.30 ppm (s, 3H, CH₃-CO); 2.53 (t, 2H, CH₂-CO); 3.08 (t, 2H, CH₂S); 3.32 (t, 4H, CH₂NHCO); 3.54 ppm (m, 8H)
- FAB-MS:: 1617.5 [M+H]⁺

### Beispiel 2:

### B₁₂-e-DADOO-(S)ATP (4a)

Umsetzung analog B₁₂-d-DADOO-(S)ATP nur mit B₁₂-e-COOH.
- Ausbeute:: 62 mg (11 %) B₁₂-e-DADOO-(S)ATP

### Analytische Daten:

- Retentionszeit analytische HPLC:: 22.6 min
- ¹H-NMR (D₂O, TMS-Na-Salz):: enthält die Signale der B₁₂-e-Carbonsäure sowie 2.33 (s, 3H, CH₃-CO); 2.65 (t, 2H, CH₂CO); 3.10 (t, 2H, CH₂S); 3.39 (t, 4H, CH₂NHCO); 3.70 ppm (m, 8H)
- ³¹P-NMR (D₂O, TMS-Na-Salz):: 1.6 ppm (s)
- FAB-MS:: 1617 [M+H]⁺

### Beispiel 3:

### B₁₂-d-Ethylendiamin-(S)ATP (4b)

500 mg (0.37 mmol) B₁₂-d-Säure werden in 30 ml absoluten DMF und 5 ml DMSO gelöst und mit 155 µl (1.09 mmol) Triethylamin versetzt. Nach 20 min Rühren bei 20°C wird das Reaktionsgemisch auf 0°C gekühlt und mit 143 µl (1.09 mmol) Chlorameisensäureisobutylester versetzt. Nach weiteren 15 min unter Eiskühlung wird eine Lösung aus 425.6 mg (1.4 mmol) Ethylendiamin-(S)ATP x Trifluoressigsäure, 193 µl (1.4 mmol) Triethylamin und 10 ml absoluten DMF zugegeben. Dabei ist eine leichte Gasentwicklung zu beobachten. Die weitere Aufarbeitung erfolgt analog der des B₁₂- DADOO-(S)ATP.
- Ausbeute:: 60 mg (11 %) B₁₂-d-ED-(S)ATP

### Analytische Daten:

- Retentionszeit analytische HPLC:: 19.0 min
- ¹H-NMR (D₂O, TMS-Na-Salz):: enthält die Signale der B₁₂-d-Carbonsäure sowie 2.30 (s, 3H, CH₃CO); 2.45 (t, 2H, CH₂CO); 2.60 (m, 2H, CH₂S); 3.05 (t, 2H, CH₂NH); 3.15 ppm (m, 2H, CH₂NH)
- FAB-MS:: 1528.6 [M+H]⁺

### Beispiel 4:

### N(tert.-Butoxycarbonyl)-1,8-diamino-3,6-dioxaoctan (6a)

### Zwischenprodukt

150 g (1 mol) 1,8-Diamino-3,6-dioxaoctan (DADOO) in 900 ml Dioxan / Wasser (v/v 1/1) gelöst werden unter Rühren langsam mit einer Lösung aus 109 g (0.5 mol) Di-tert.-butyldicarbonat in 450 ml Dioxan versetzt. Nach dem Zutropfen wird das Reaktionsgemisch noch 1.5 h bei 20°C gerührt, anschließend das Lösungsmittel abdestilliert und der Rückstand in 1000 ml Essigester/Wasser (v/v 1/1) aufgenommen. Nach dem Abtrennen der wässrigen Phase extrahiert man die organische Phase zweimal mit je 500 ml 0.1 n Salzsäure. Die wässrigen Phasen werden vereinigt, der pH-Wert mit verdünnter Natronlauge auf pH 9-10 einstellt und die Lösung einer Flüssig-Flüssig-Extraktion im Perforator unterworfen. Nach 8-stündigem Extrahieren mit 750 ml Essigester wird das Lösungsmittel entfernt und der Rückstand am HV getrocknet.
- Ausbeute:: 58.2 g (23.5 % d. Th.)
- ¹H-NMR (CDCl₃/TMS):: 1.45 (s, 9H, O-C(CH₃)₃); 2.88 (t, 2H, J=5.3 Hz, CH₂NH₂); 3.28 (t, 2H, J=5.7 Hz, CH₂NH); 3.52 (m, 4H, CH₂O); 3.57 (s, 4H, CH₂O); 5.21 ppm (s, br, 1H, NHCO)
- DC:: Kieselgel 60 (Merck),
Isopropanol/Butylacetat/Wasser/Ammoniak (v/v/v/v 50/30/15/5),
Detektion mit Ninhydrin.
R_{f} = 0.45

### Beispiel 5:

### 1-Butoxycarbonyl-1,2-diaminoethan (m-Boc-ED) (6b)

### Zwischenprodukt

Zu einer Lösung aus 120 g (2 mol) Ethylendiamin und 2 l Dioxan/Wasser (v/v 1/1) werden unter Rühren bei 0°C 218 g (1 mol) Di-tert.butyldiarbonat in 1 l Dioxan innerhalb 1 h zugetropft. Nach einstündigem Rühren bei 20°C engt man das Lösungsmittel im Wasserstrahlvakuum ein, filtriert vom als Nebenprodukt entstandenen Bis-1,2-butoxycarbonyl-1,2-diaminoethan ab und extrahiert das Filtrat mit 300 ml Essigester. Die organische Phase wird noch dreimal mit je 100 ml Wasser gewaschen, über Natriumsulfat getrocknet und im Wasserstrahlvakuum eingeengt.
- Ausbeute:: 27.3 g (8.5 % d. Th.)
- ¹H-NMR (CDCl₃/TMS):: 1.45 (s, 9H, O-C(CH₃)₃); 2.79 (T, 2H, J=7.5 Hz, CH₂-NH); 3.14 (m, 4H, CH₂-NH₂); 5.00 ppm (s, br, 1H, O-CONH)
- DC:: Kieselgel 60 (Merck), Isopropanol/Butylacetat/Wasser/Ammoniak (v/v/v/v/ 50/30/15/5), Detektion mit Ninhydrin.
R_{f} = 0.23

### Beispiel 6:

### 1-Butoxycarbonyl-8-[(3-methylcarbonylthio)propionyl]-1,8-diamino-3,6-dioxaoktan (Boc-DADOO-(S)ATP) (8a)

12.41 g (50 mmol) 6a und 12.26 g (50 mmol) N-Succinimidyl-S-acetylthiopropionat (SATP) werden in 100 ml Tetrahydrofuran gelöst und 4 h bei 20°C gerührt. Anschließend wird das Lösungsmittel abdestilliert, der Rückstand in 250 ml Essigester/ Wasser (v/v 1/1) aufgenommen und mit 6 n HCl auf pH 4-5 eingestellt. Die organische Phase wird abgetrennt, zweimal mit je 100 ml Wasser extrahiert, über Natriumsulfat getrocknet, das Lösungsmittel abdestilliert und der Rückstand im Hochvakuum getrocknet.
- Ausbeute:: 11.97 g ( 63.2 % d. Th.)
- ¹H-NMR (CDCl₃/TMS):: 1.45 (s, 9H, O-C(CH₃)₃); 2.33 (s, 3H, CH₃); 2.51 (t, 2H, J=7.0 Hz, CH₂CO); 3.14 (t, 2H, J=7.0 Hz,CH₂S); 3.56 (m, 12H); 5.39 (s br, 1H, NH-COO); 6.79 ppm (s br, 1H, NH-CO)
- IR (Film):: 3300, 2980, 1740, 1680, 1520 cm⁻¹

### Beispiel 7:

### 1-Butoxycarbonyl-2-[(3-methylcarbonylthio)propionyl]-1,2-diamino-ethan (Boc-ED-(S)ATP) (8b)

8.00 g (50 mmol) 6b und 12.26 g (50 mmol) N-Succinimidyl-S-acetylthiopropionat (SATP) werden in 100 ml Tetrahydrofuran gelöst und 4 h bei 20°C gerührt. Anschließend kühlt man die Lösung im Eisbad, saugt das ausgefallene Produkt ab und trocknet es im Hochvakuum bei 40°C.
- Ausbeute:: 9.22 g (63.9 % d. Th.)
- ¹H-NMR (CDCl₃/TMS):: 1.44 (s, 9H, O-C(CH₃)₃); 2.32 (s, 3H, - CH₃); 2.52 (t, 2H, J=7.0 Hz, CH₂CO); 3.14 (t, 2H, J=7.0 Hz, CH₂S); 3.33 (m, 4H, CH₂-NH); 5.00 (s, br, 1H, OCONH); 6.37 ppm (s, br, 1H, CONH)
- DC:: Kieselgel 60 (Merck), Isopropanol/Butylacetat/Wasser/Ammoniak (v/v/v/v 50/30/15/5), Detektion mit Ninhydrin.
R_{f} = 0.87

### Beispiel 8:

### 1-[(3-Methylcarbonylthio)propionyl]-1,8-diamino-3,6-dioxaoctan- Trifluoracetat (DADOO-(S)ATP x CF₃COOH) (3a)

9.46 g (25 mmol) 8a werden in 50 ml Trifluoressigsäure gelöst und bei 0°C gerührt. Nach beendeter Gasentwicklung wird der Reaktionsansatz langsam auf 20°C gebracht und das Lösungsmittel am Wasserstrahlvakuum abdestilliert. Der ölige Rückstand wird im Hochvakuum getrocknet.
- Ausbeute:: 9.41 g (100 % d. Th.)
- ¹H-NMR (CDCl₃/TMS):: 2.30 (s, 3H, CH₃); 2.52 (t, 2H, J=7.0 Hz, CH₂-CO); 3.08 (t, 2H, J=7.0 Hz, CH₂-S); 3.62 (m, 12H); 7.05 (s br, 1H, NH-CO); 7.64 ppm (s br, 3H, NH)
- DC:: Kieselgel 60 (Merck), Essigester/Eisessig/Wasser (v/v/v 5/5/2), Detektion mit Ninhydrin.
R_{f} = 0.58

### Beispiel 9:

### 1-[(3-Methylcarbonylthio)propionyl]-1,2-diaminoethan-Trifluoracetat (ED-(S)ATP x TFA) (3b)

7.25 g (25 mmol) 8b werden in 10 ml Trifluoressigsäure gelöst und 4 h bei 20°C gerührt. Anschließend wird die Lösung im Wasserstrahlvakuum zur Trockne eingedampft und der Rückstand mit 50 ml Diisopropylether digeriert. Das Produkt wird im Hochvakuum getrocknet und kristallisiert nach einer längeren Induktionsphase aus.
- Ausbeute:: 5.54 g (65 % d. Th.)
- ¹H-NMR (D₆-DMSO/TMS):: 2.31 (S, 3H, -CH₃); 2.39 (t, 2H, J=7.0 Hz, CH₂-CO); 2.85 (t, 2H, J=7.0 Hz, CH₂S); 3.00 (t, 2H, J=7.0 Hz, CH₂NH); 3.26 (t,2H, J=7.0 Hz, CH₂NH); 7.92 (s, br, NH); 8.13 ppm (s, br, 1H, NH-CO)
- DC:: Kieselgel 60 (Merck), Essigester/Eisessig/Wasser (v/v/v 5/5/2), Detektion mit Ninhydrin.
R_{f} = 0.62

### Beispiel 10:

### B₁₂-d-DADOO und B₁₂-e-DADOO (9)

500 mg (0.37 mmol) der entsprechenden B₁₂-Monocarbonsäure werden in 30 ml absoluten DMF und 5 ml DMSO gelöst und mit 155 µl (1.09 mmol) Triethylamin versetzt. Nach 20 min Rühren bei 20°C wird das Reaktionsgemisch auf 0°C gekühlt und mit 143 µl (1.09 mmol) Chlorameisensäureisobutylester versetzt. Nach weiteren 15 min unter Eiskühlung werden 548 mg (3.7 mmol) Diaminodioxaoktan zugegeben. Dabei ist eine leichte Gasentwicklung zu beobachten. Das weitere Vorgehen ist analog der Umsetzung mit DADOO-(S)ATP.
- Ausbeute:: 49 mg (9 %) B₁₂-d-DADOO
160 mg (29 %) B₁₂-e-DADOO

### Analytische Daten B₁₂-d-DADOO:

- Retentionszeit der analytischen HPLC:: 19.5 min
- IR (KBr):: γ = 3350, 3170, 2120, 1660, 1570, 1495, 1398 cm⁻¹
- ¹H-NMR (D₂O, TMS-Na-Salz):: enthält die Signale der B₁₂-d-Carbonsäure sowie 3.20 (t, 4H), 3.70 ppm (m, 8H)
- FAB-MS:: 1486.6 [M]⁺

### Analytische Daten B₁₂-e-DADOO:

- Retentionszeit der analytischen HPLC:: 19.5 min
- ¹H-NMR (D₂O, TMS-Na-Salz):: enthält die Signale der B₁₂-e-Carbonsäure sowie 3.22 (2t, 4H), 3.73 ppm (m, 8H)
- ³¹P-NMR (D₂O, TMS-Na-Salz):: 1.48 (s)
- FAB-MS:: 1486.9 [M+H]⁺

### Beispiel 11:

### B₁₂-d-DADOO-(S)ATP (4a)

5 mg (3.7 x 10 ⁻³ mmol) B₁₂-d-DADOO (9) werden in 1 ml DMF gelöst, mit einer Spatelspitze SATP, ebenfalls in 1 ml DMF gelöst, versetzt und 5 h bei 20°C gerührt. Die Aufreinigung erfolgt mittels HPLC.

### Analytische Daten:

- Retentionszeit:: 22.3 min

### Beispiel 12:

### Biotin-DADOO-(S)ATP (14)

400 mg (1.07 mmol) Biotin-DADOO werden in 10 ml DMF gelöst, mit 262 mg (1.07 mmol) SATP versetzt und 4 d bei 4°C gerührt. Nach dem Entfernen des Lösungsmittels wird der Rückstand mit Essigester digeriert, abgesaugt und getrocknet. Die Reinigung des Rohprodukts erfolgt mittels offener Säulenchromatographie (SiO₂, Methanol/Essigester (v/v 1/1).

### Analytische Daten:

- Retentionszeit:: Biotin-DADOO-(S)ATP: 22.10 min
- ¹H-NMR (D₆-DMSO, TMS):: 1.40 (m, 6H, 3 CH₂); 2.06 (t, 2H, CH₂CO); 2.31 (s, 3H, CH₃CO); 2.37 (t, 2H, CH₂CO); 2.77 (d, 1H, CHS); 2.99 (t, 2H, CH₂S); 3.16-3.70 (m, 14H); 4.22 (m, 2H, 2 CHNH); 6.40 (d, 2H, 2 NH); 7.85 (t, 1H, NHCH₂); 8.01 ppm (t, 1H, NHCH₂)

### Beispiel 13:

### B₁₂-d-MEA (13)

50 mg (0.037 mmol) B₁₂-d-Säure werden in 3 ml DMF und 2 ml DMSO gelöst und mit 16 µl (0.109 mmol) Triethylamin versetzt. Nach 15 min Rühren bei 20°C wird der Ansatz auf 0°C abgekühlt, mit 14 µl (0.109 mmol) Chlorameisensäureisobutylester versetzt und weitere 20 min bei 0°C gerührt. Anschliessend wird eine Lösung von 19.5 mg (0.111 mmol) Maleinimidoethylamin-Hydrochlorid (MEA x HCl) in 3 ml DMF/Triethylamin 2/1 (v/v) zugetropft und 1 h bei 20°C gerührt. Das Rohprodukt wird ohne weitere Aufreinigung der Folgereaktion unterworfen.

### Analytische Daten:

- Retentionszeiten:: MEA x HCl: 9.81 min
B₁₂-d-COOH: 11.80 min
B₁₂-d-MEA: 20.33 min

### Beispiel 14:

### B₁₂-d-MEA-(SA)TP-DADOO-Biotin (15)

30 mg (0.059 mmol) Biotin-DADOO-(S)ATP 2 werden in 5 ml 0.025 M Hydroxylaminlösung gelöst und 1 h unter Argonatmosphäre bei 20°C gerührt. Das Lösungsmittel wird im Wasserstrahlvakuum eingeengt und der Rückstand in 2 ml DMF aufgenommen. Diese Lösung wird mit der Reaktionslösung 13 vereinigt und 1 h bei 20°C gerührt. Nach Abtrennung des Lösungsmittels und Entsalzen über Amberlite®XAD 2 erfolgt die Reinigung des Produkts mittels präparativer HPLC.

### Analytische Daten:

- Retentionszeit:: Biotin-DADOO-(SA)TP: 19.95 min
B₁₂-d-MEA-(SA)TP-DADOO-Biotin: 23.05 min

### Beispiel 15:

### Konjugation von B₁₂-d-DADOO-(S)ATP an vorpolymerisierte Peroxidase (pPOD)

Vorpolymerisierte POD kann beispielsweise mit Glutardialdehyd, wie bei Engvall und Perlmann beschrieben
(Immunochemistry 8 (1971), 871-874), erhalten werden.

### a) Aktivierung von pPOD mit Maleinimidohexanoyl-N-hydroxysuccinimidester (MHS)

Die Einführung von Maleinimidgruppen in pPOD erfolgt nach Yoshitake, Eur.J.Biochem. 101, 395-399 (1979):
MHS wird unmittelbar vor Verwendung in DMSO frisch gelöst (160 mg/ml DMSO). pPOD wird auf eine Proteinkonzentration von 25 mg/ml in 0,04 mol/l Kaliumphosphatpuffer pH 7,1 eingestellt. Je 30 µl der MHS-Lösung werden zu je 1 ml der pPOD-Lösung pipettiert, so daß ein molares POD:MHS-Verhältnis von 1:25 erreicht wird. Nach 1-stündiger Inkubation des resultierenden Gemisches bei 25°C werden je ml Reaktionsansatz 10 µl einer Lysinlösung (1 mol/l) zupipettiert und weitere 30 Minuten bei 25°C inkubiert. Die aktivierte pPOD-Lösung (pPOD-MH) wird über Nacht unter Kühlung gegen ein 1000-faches Volumen vorgekühlten Puffer (100 mmol/l Kaliumphosphat/1 mmol/l EDTA/pH 6,0) dialysiert. Nach Dialyse wird pPOD-MH auf eine Proteinkonzentration von 10 mg/ml eingestellt.

### b) Aktivierung von B₁₂-d-DADOO-(S)ATP

20 mg B₁₂-d-DADOO-(S)ATP werden in 4 ml H₂O mit 40 µl Hydroxylaminlösung (2 mmol/l pH 6,3) 20 Minuten bei 25°C inkubiert.

### c) Konjugation von B₁₂-d-DADOO-(S)ATP an pPOD-MH

5 ml der pPOD-MH-Lösung (50 mg pPOD-MH) werden mit 400 µl der aktivierten B₁₂-d-DADOO-(S)ATP-Lösung 3 Stunden unter Rühren bei 25°C inkubiert. Zum Stoppen der Reaktion werden 10 µl einer Cysteinlösung (1 mol/l in H₂O) zupipettiert, so daß eine Cysteinkonzentration von 2 mmol/l resultiert. Nach einer weiteren 30 minütigen Inkubation bei 25°C werden 270 µl einer 100 mmol/l N-Methylmaleinimid-Lösung (NMM) in DMSO (11 mg/ml) zupipettiert und so eine NMM-Konzentration von 5 mmol/l eingestellt und weitere 60 Minuten bei 25°C inkubiert.

Der Konjugationsansatz wird zur Aufreinigung über eine Sephadex®G-25 coarse Säule chromatographiert. Als Laufpuffer wird 40 mmol/l Kaliumphosphatpuffer, pH 6,0 eingesetzt.

Das gereinigte B₁₂-d-DADOO-S-pPOD-Konjugat wird auf eine Peroxidase-Aktivität von ca. 2000 U/ml konzentriert.

### Beispiel 16:

### Bestimmung von Vitamin B₁₂

Die Bestimmung von Vitamin B₁₂ wird analog zur Beschreibung in EP-A 0 378 197 durchgeführt.

### a) Probenvorbereitung

Die Probenvorbereitung, d.h. die Ablösung des B₁₂ von seinem Bindeprotein, kann analog EP-A 0 378 204 durch Behandlung mit Liponsäure oder durch Hitzeeinwirkung oder durch Zerstörung des Bindeproteins im alkalischen Bereich (pH > 13,5) durchgeführt werden.

250 µl Humanserum werden mit 125 µl Ablösereagenz (bestehend aus 8 mg/ml Liponsäure, 1 mg/ml Kaliumcyanid gelöst in 0,5 mol/l NaOH) gemischt und 15 Minuten bei Raumtemperatur inkubiert. Anschließend werden 125 µl einer Phosphatpufferlösung (200 mmol/l; pH 4,1) zugegeben.

### b) Reagenzien

Polystyrolröhrchen beschichtet mit Thermo-RSA Streptavidin (hergestellt nach EP-A 0 269 092)

### Reagenz 1

Biotinylierter monoklonaler Antikörper (MAK-BI) aus EP-A 0 378 197, biotinyliert nach JACS 100 (1978), 3585-3590; 40 mmol/l Phosphatpuffer, pH 7,2.
MAK-BI Konzentration in Verbindung mit Reagenz 2a: 6 ng/ml
MAK-BI Konzentration in Verbindung mit Reagenz 2b: 75 ng/ml

### Reagenz 2a

### B₁₂-d-DADOO-S-pPOD

Aktivität: 500 mU/ml
   20 mmol/l Na-Phosphatpuffer;
   10 mmol/l EDTA; 150 mmol/l NaCl; pH 6,8
   0,25 % Rinderserumalbumin (RSA); 0,05 % Tween® 20

### Reagenz 2b (Vergleichskonjugat nach EP-A 0 378 203)

### B₁₂-d-CO-NH-NH-CO-CH₂-(-O-CH₂-CH₂-)₃-O-CH₂-CO-NH-N=POD (= B₁₂-TEDEH-POD)

Aktivität: 30 mU/ml.
   20 mmol/l Na-Phosphatpuffer;
   10 mmol/l EDTA; 150 mmol/l NaCl; pH 6,8
   0,25 % RSA, 0,05 % Tween® 20

### Reagenz 3

100 mmol/l Phosphat-Citratpuffer, pH 4,4.
1,9 mmol/l ABTSs
3,2 mmol/l Natriumperborat.

### c) Durchführung

Zur Durchführung der Bestimmung werden 100 µl vorbehandelte Probe- bzw. Standardlösung mit 500 µl Reagenz 1 (MAK-Lösung) in ein Streptavidintube pipettiert und 30 Minuten bei 25°C inkubiert. Anschließend werden 500 µl Reagenz 2 (Konjugat-Lösung) zupipettiert und weitere 60 Minuten bei 25°C inkubiert. Es wird mit Waschlösung gewaschen und 1000 µl Reagenz 3 (Substrat-Lösung) zugegeben, für 30 Minuten bei 25°C inkubiert und die gebildete Farbe als Maß für Vitamin B₁₂-Gehalt bei 422 nm gemessen.

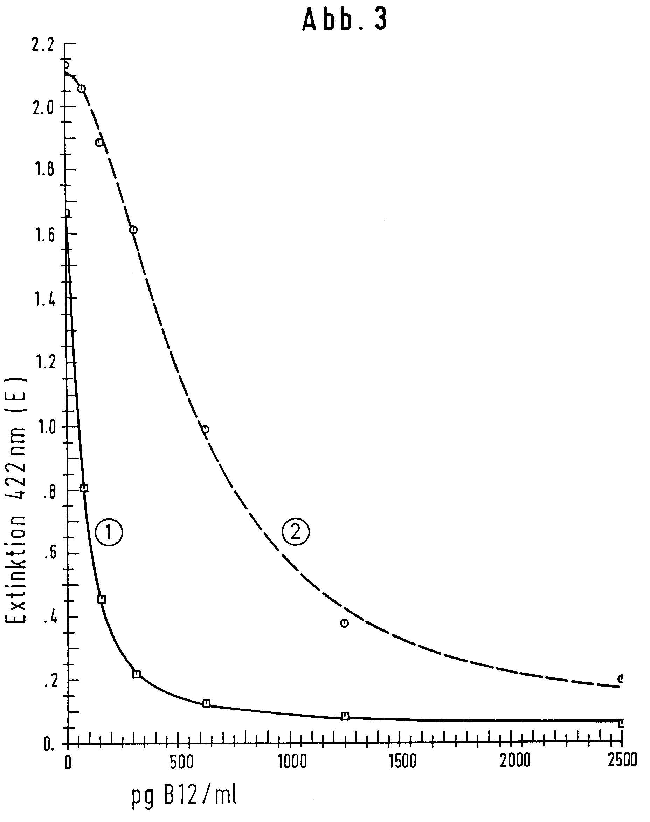

Abb. 3 zeigt den Vergleich von B₁₂-Eichkurven, die mit dem Konjugat entsprechend der Erfindung (Reagenz 2a, Kurve 1) und einem Konjugat des Standes der Technik (Reagenz 2b, Kurve 2) erhalten wurden.

Danach zeigt sich, daß mit dem erfindungsgemäßen Konjugat steilere und damit bessere Eichkurven erzielt werden.

## Patentansprüche

1. Verfahren zur Herstellung von Cobalamin-Konjugaten der Formel (I)
B - CO - Sp - P (I)
wobei B den aus einem Cobalamin durch Abspaltung einer -CONH₂ Gruppe gebildeten Rest,
P ein nachweisbares Molekül als Kupplungspartner des Cobalamins und Sp eine Spacergruppierung darstellt,
**dadurch gekennzeichnet,**
daß man eine Cobalamin-Monocarbonsäure B-COOH mit einem Chlorameisensäureester der allgemeinen Formel (II) 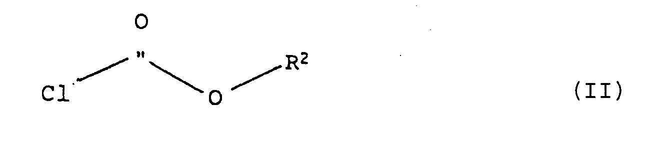 als Kondensationsmittel zur Kupplung umsetzt, worin R² einen geradkettigen oder verzweigten Alkylrest mit 1 bis 6 Kohlenstoffatomen darstellt, wobei eine aktivierte Cobalamin-Monocarbonsäure der allgemeinen Formel (III) 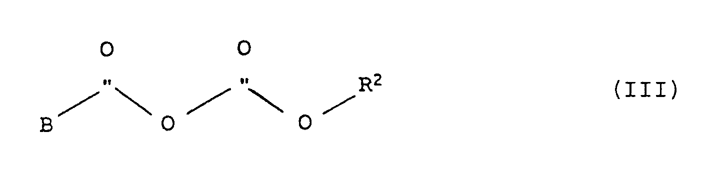 entsteht, die anschließend als Ausgangsmaterial für die Verknüpfung mit dem Kupplungspartner P über den Spacer Sp dient.

2. Verfahren nach Anspruch 1,
**dadurch gekennzeichnet,**
daß das Cobalamin die allgemeine Formel (IV) 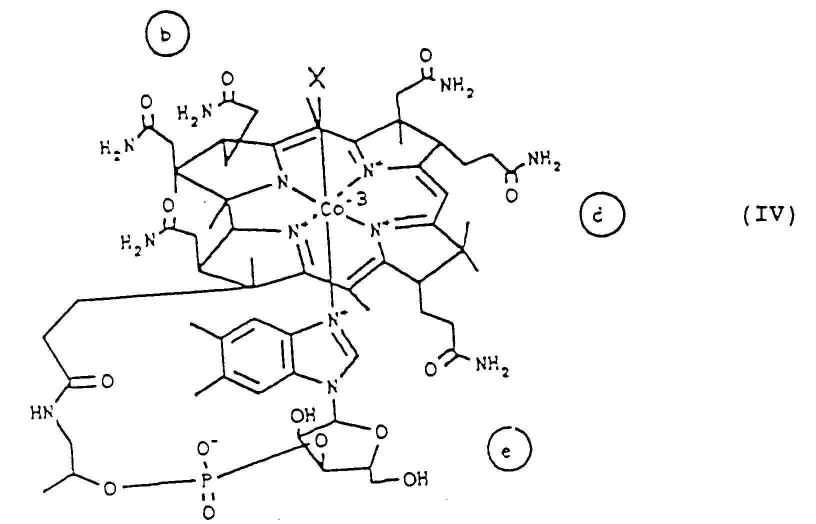 aufweist, wobei X einen Liganden des Kobalts darstellt und die Bindung des Cobalamins mit der Spacergruppierung über eine der mit (b), (d) oder (e) bezeichneten Positionen erfolgt.

3. Verfahren nach Anspruch 1 oder 2,
**dadurch gekennzeichnet,**
daß man eine aktivierte Cobalamin-Monocarbonsäure der allgemeinen Formel (III) mit einem Maleimid-Derivat der allgemeinen Formel (XIII) 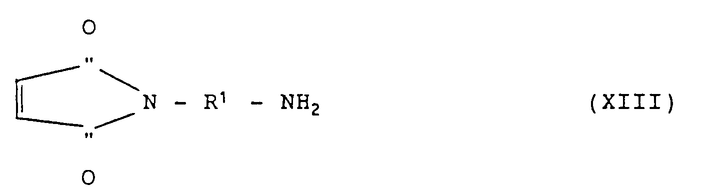 umsetzt, wobei R¹ eine unverzweigte Alkylengruppe mit 2 bis 20 Kohlenstoffatomen darstellt, die durch ein oder mehrere Heteroatome, insbesondere Sauerstoff- oder Schwefelatome, substituiert sein kann, das entstehende Produkt der allgemeinen Formel (XIV) 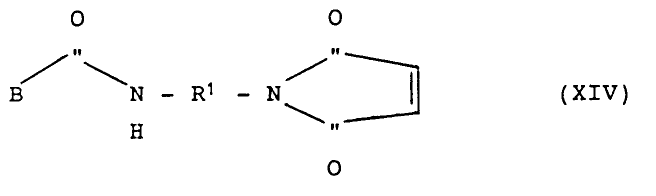 mit der SH-Funktion eines Thiol-funktionalisierten Kupplungspartners HS ∼ P umsetzt, wobei als Endprodukt ein Cobalamin-Konjugat der allgemeinen Formel 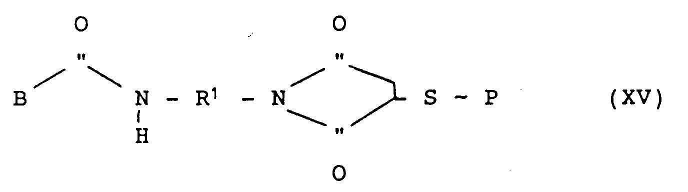 entsteht.

4. Cobalamin-Konjugat mit der allgemeinen Formel (XVII) 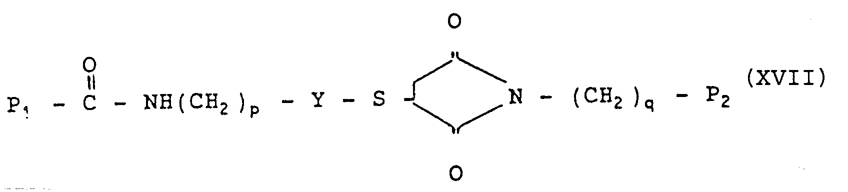 worin
entweder P₁ einen aus einem Cobalamin durch Abspaltung einer -CONH₂-Gruppe gebildeten Rest und P₂ ein nachweisbares Molekül als Kupplungspartner des Cobalamins darstellt,
oder P₂ einem aus einem Cobalamin durch Abspaltung eines H-Atoms gebildeten Rest und P₁ ein nachweisbares Molekül als Kupplungspartner des Cobalamins darstellt,
p und q gleich oder verschieden und ganze Zahlen von 2 bis 6 sind, und
Y eine Einfachbindung oder eine Gruppe der allgemeinen Formel (XVIII) 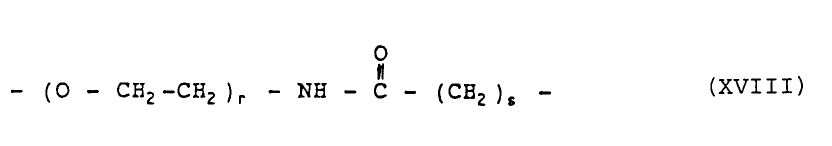 ist, wobei r eine ganze Zahl von 0 bis 3 und s eine ganze Zahl von 1 bis 3 ist.

5. Konjugat nach Anspruch 4,
**dadurch gekennzeichnet,**
daß das Cobalamin die allgemeine Formel (IV) gemäß Anspruch 2 besitzt.

6. Konjugat nach Anspruch 5,
**dadurch gekennzeichnet,**
daß die Bindung über eine der mit (d) oder (e) bezeichneten Positionen erfolgt.

7. Konjugat nach Anspruch 5 oder 6,
**dadurch gekennzeichnet,**
daß X eine Cyanogruppe ist.

8. Konjugat nach einem der Ansprüche 5 bis 7,
**dadurch gekennzeichnet,**
daß der Kupplungspartner des Cobalamins eine Peroxidase, β-Galactosidase, alkalische Phosphatase oder ein Streptavidin ist.

9. Konjugat nach einem der Ansprüche 5 bis 7,
**dadurch gekennzeichnet,**
daß der Kupplungspartner des Cobalamins Biotin ist.

10. Verwendung von Cobalamin-Konjugaten nach einem der Ansprüche 4 bis 9 in Immuntests, insbesondere zur Bestimmung von Cyanocobalamin.

11. Verwendung nach Anspruch 10 in einem ELISA-Test.

12. Verfahren nach einem der Ansprüche 1 bis 3, weiterhin umfassend die Verwendung der hergestellte Cobalamin-Konjugate in Immuntests, insbesondere zur Bestimmung von Cyanocobalamin.

## Claims

1. Process for the production of cobalamin conjugates of formula (I)
B - CO - Sp - P (I)
in which B represents the residue formed from a cobalamin by cleavage of a -CONH₂ residue,
P represents a detectable molecule as coupling partner of the cobalamin and Sp represents a spacer group,
**wherein**
a cobalamin monocarboxylic acid B-COOH is reacted with a chloroformic acid ester of the general formula (II) 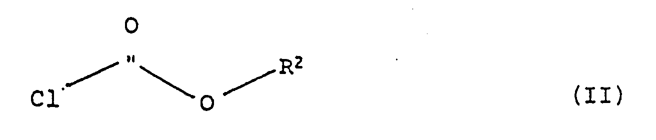 as the condensing agent for the coupling, in which R² represents a straight-chained or branched alkyl residue with 1 to 6 carbon atoms, thereby forming an activated cobalamin monocarboxylic acid of the general formula (III) 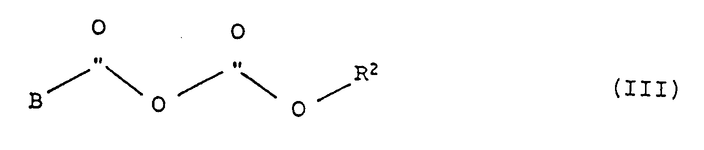 which subsequently serves as the starting material for linkage to the coupling partner P via the spacer Sp.

2. Process as claimed in claim 1,
**wherein**
the cobalamin has the general formula (IV) 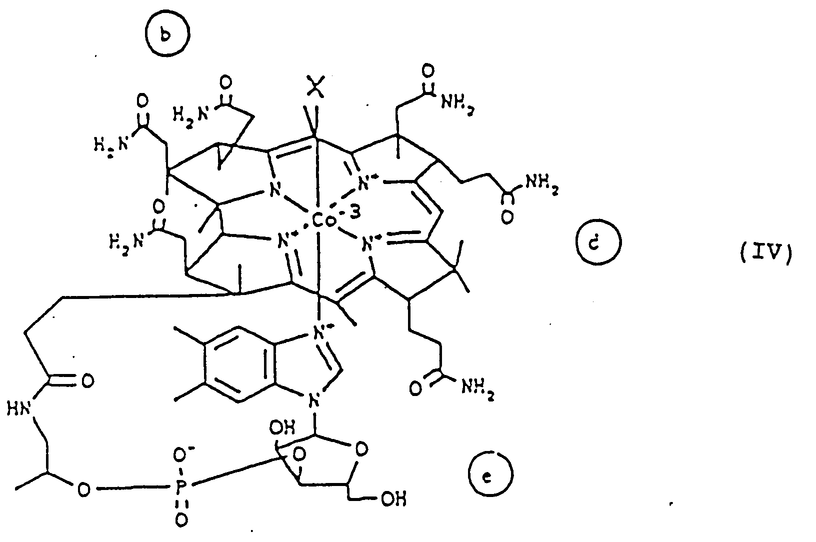 in which X represents a ligand of cobalt and the cobalamin is linked to the spacer group via one of the positions denoted (b), (d) or (e).

3. Process as claimed in claim 1 or 2,
**wherein**
the activated cobalamin monocarboxylic acid of the general formula (III) is reacted with a maleimide derivative of the general formula (XIII) 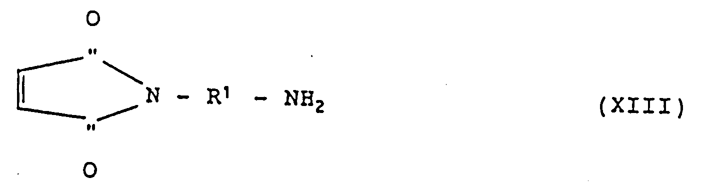 in which R¹ represents an unbranched alkylene group with 2 to 20 carbon atoms which can be substituted by one or several heteroatoms, in particular by oxygen or sulphur atoms, the product which forms of the general formula (XIV) 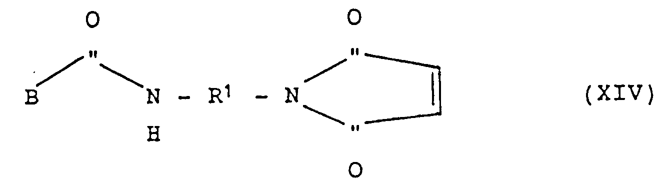 is reacted with the SH group of a coupling partner HS ∼ P provided with a thiol functional group to form a cobalamin conjugate of the general formula

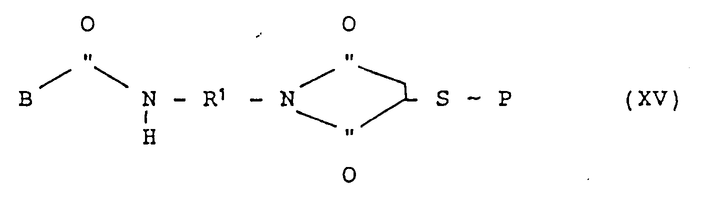

4. Cobalamin conjugate having the general formula (XVII) 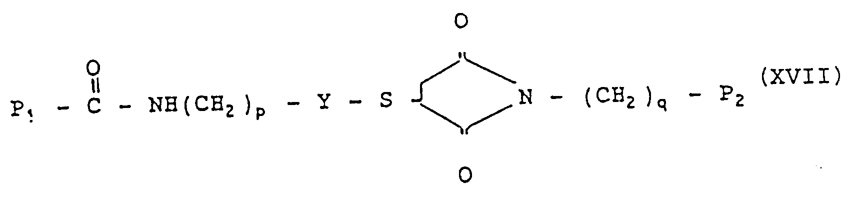 in which
P₁ either represents a residue formed from a cobalamin by cleavage of a -CONH₂ residue and P₂ represents a detectable molecule as the coupling partner of the cobalamin or P₂ represents a residue formed from a cobalamin by cleavage of a H atom and P₁ represents a detectable molecule as the coupling partner of the cobalamin,
p and q are the same or different and are integers from 2 to 6 and
Y is a single bond or a group of the general formula (XVIII) 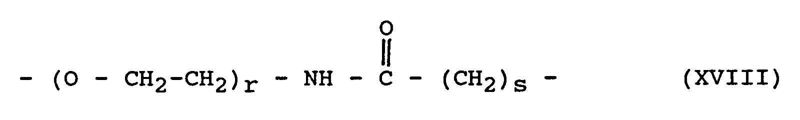 in which r is an integer from 0 to 3 and s is an integer from 1 to 3.

5. Conjugate as claimed in claim 4,
**wherein**
the cobalamin has the general formula (IV) according to claim 2.

6. Conjugate as claimed in claim 5,
**wherein**
binding takes place via one of the positions denoted (d) or (e).

7. Conjugate as claimed in claim 5 or 6,
**wherein**
X is a cyano group.

8. Conjugate as claimed in one of the claims 5 to 7,
**wherein**
the coupling partner of the cobalamin is a peroxidase, β-galactosidase, alkaline phosphatase or streptavidin.

9. Conjugate as claimed in one of the claims 5 to 7,
**wherein**
the coupling partner of the cobalamin is biotin.

10. Use of cobalamin conjugates as claimed in one of the claims 4 to 9 in immunological tests, in particular for the determination of cyanocobalamin.

11. Use as claimed in claim 10 in an ELISA test.

12. Process as claimed in one of the claims 1 to 3 additionally comprising the use of the produced cobalamin conjugates in immunological tests, in particular for the determination of cyanocobalamin.

## Revendications

1. Procédé de préparation de conjugués de cobalamine de formule (I)
B - CO - Sp - P (I)
où B représente le reste formé à partir d'une cobalamine par clivage d'un groupe -CONH₂,
P représente une molécule qui peut être mise en évidence comme partenaire de couplage de la cobalamine et Sp représente une groupement espaceur,
caractérisé en ce que l'on fait réagir un acide cobalaminemonocarboxylique B-COOH avec un ester d'acide chloroformique de formule générale (II) 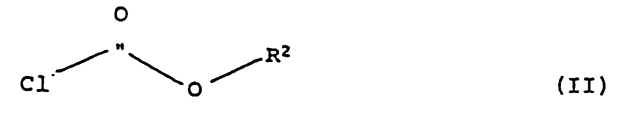 comme agent de condensation pour le couplage, où R² représente un reste alkyle linéaire ou ramifié de 1 à 6 atomes de carbone, de sorte qu'il se forme un acide cobalamine-monocarboxylique activé de formule générale (III) 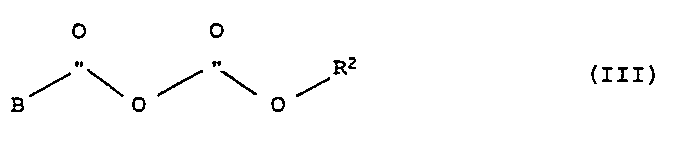 qui sert ensuite de produit de départ pour la liaison avec le partenaire de couplage P par l'intermédiaire de l'espaceur Sp.

2. Procédé selon la revendication 1 caractérisé en ce que la cobalamine présente la formule générale (IV) 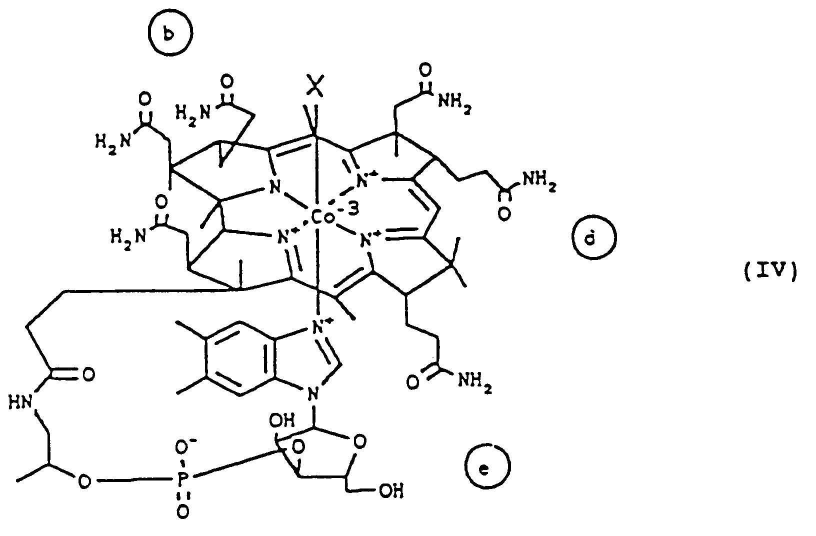 où X représente un ligand du cobalt et la liaison de la cobalamine avec le groupement espaceur a lieu par l'intermédiaire de l'une des positions désignées par (b), (d) ou (e).

3. Procédé selon la revendication 1 ou 2 caractérisé en ce que l'on fait réagir un acide cobalaminemonocarboxylique activé de formule générale (III) avec un dérivé du maléimide de formule générale (XIII) 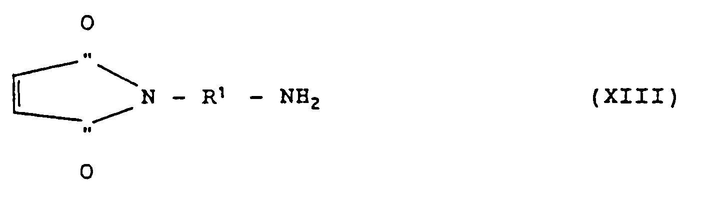 où R¹ représente un groupe alkylène non ramifié de 2 à 20 atomes de carbone qui peut être substitué par un ou plusieurs hétéroatomes, en particulier des atomes d'oxygène ou de soufre, on fait réagir le produit formé de formule générale (XIV) 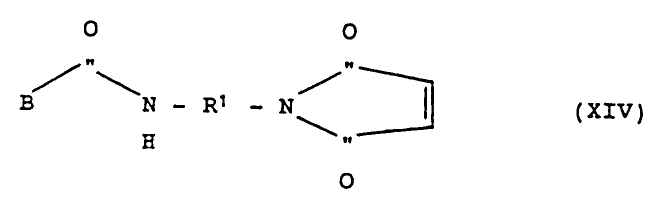 avec la fonction SH d'un partenaire de couplage à fonctionnalité thiol HS ∼ P, de sorte qu'un conjugué de cobalamine de formule générale 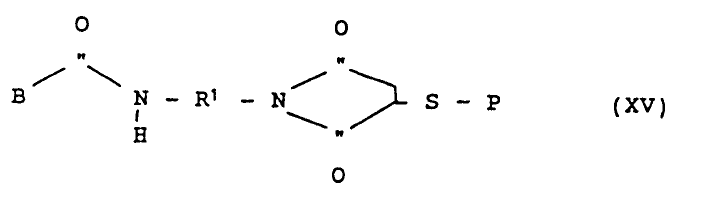 se forme comme produit final.

4. Conjugué de cobalamine de formule générale (XVII) 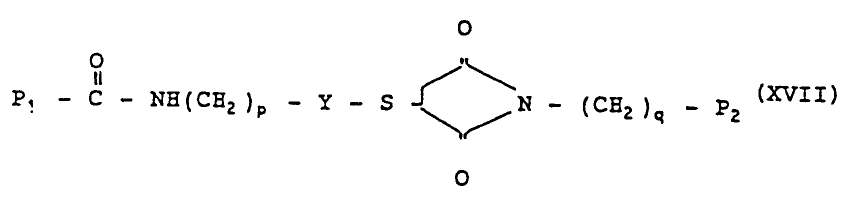 où
P₁ représente un reste formé à partir d'une cobalamine par clivage d'un groupe -CONH₂ et P₂ représente une molécule qui peut être mise en évidence comme partenaire de couplage de la cobalamine, ou P₂ représente un reste formé à partir d'une cobalamine par clivage d'un atome H et P₁ représente une molécule qui peut être mise en évidence comme partenaire de couplage de la cobalamine,
p et q sont identiques ou différents et sont des nombres entiers de 2 à 6, et
Y représente une simple liaison ou un groupe de formule générale (XVIII) 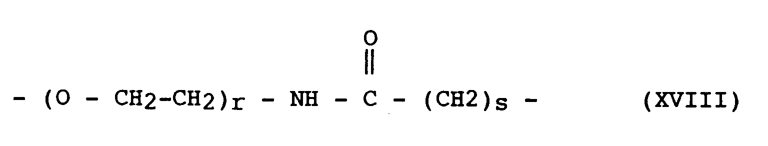 où r est un nombre entier de 0 à 3 et s est un nombre entier de 1 à 3.

5. Conjugué selon la revendication 4 caractérisé en ce que la cobalamine possède la formule générale (IV) selon la revendication 2.

6. Conjugué selon la revendication 5 caractérisé en ce que la liaison a lieu par l'intermédiaire de l'une des positions désignées par (d) ou (e).

7. Conjugué selon la revendication 5 ou 6 caractérisé en ce que X est un groupe cyano.

8. Conjugué selon l'une des revendications 5 à 7 caractérisé en ce que le partenaire de couplage de la cobalamine est une peroxydase, β-galactosidase, phosphatase alcaline ou une streptavidine.

9. Conjugué selon l'une des revendications 5 à 7 caractérisé en ce que le partenaire de couplage de la cobalamine est la biotine.

10. Utilisation de conjugués de cobalamine selon l'une des revendications 4 à 9 dans des immunotests, en particulier pour la détermination d'une cyanocobalamine.

11. Utilisation selon la revendication 10 dans un test ELISA.

12. Procédé selon l'une des revendications 1 à 3 comprenant en outre l'utilisation des conjugués de cobalamine préparés dans des immunotests, en particulier pour la détermination d'une cyanocobalamine.
